(19) Europäisches Patentamt
European Patent Office
Office européen des brevets

(11) **EP 3 590 952 A1**

(12) **EUROPEAN PATENT APPLICATION**
published in accordance with Art. 153(4) EPC

(43) Date of publication:
**08.01.2020 Bulletin 2020/02**

(51) Int Cl.:
*C07K 2/00* (2006.01)     *C12N 1/04* (2006.01)
*C12N 11/08* (2006.01)     *C12N 15/09* (2006.01)

(21) Application number: **18760911.0**

(22) Date of filing: **02.03.2018**

(86) International application number:
**PCT/JP2018/007943**

(87) International publication number:
**WO 2018/159797 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.03.2017   JP 2017038938**

(71) Applicant: **Fujifilm Corporation**
**Minato-ku**
**Tokyo 106-8620 (JP)**

(72) Inventors:
• **NAKAMURA, Kentaro**
**Ashigarakami-gun**
**Kanagawa 258-8577 (JP)**
• **YOSHIOKA, Yasuhiro**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(54) **EMBEDDING AGENT FOR CELL MASS OR CELL STRUCTURE, AND CELL MASS- OR CELL STRUCTURE-CONTAINING COMPOSITION AND KIT**

(57)     The object of the present invention is to provide a cell mass or cell structure-embedding agent that can stably transport cell masses or cell structures in a case of transportation at a low temperature and after transportation, conveniently recover a cell mass or cell structure from the cell mass or cell structure-embedding agent, a cell mass or cell structure-containing composition, and a kit. According to the present invention, provided is a cell mass or cell structure-embedding agent including: polypeptide which is represented by Formula 1 and in which an area of the maximum molecular weight peak in molecular weight distribution measurement is 80% or more of the total area of all of the molecular weight peaks.

Formula (1):          A-[(Gly-X-Y)n]m-B

In the formula, X's and Y's each independently represent an amino acid, m is an integer of 2 to 10, n is an integer of 3 to 100, and A and B represent any amino acids or amino acid sequences.

SEQUENCE LISTING
<110> FUJIFILM Corporation
<120> A cell mass or cell construct-embedding agent, a cell mass or cell construct-containing composition and a kit
<130> \201@17F02612
<160> 10
<170> PatentIn version 3.5
<210> 1
<211> 571
<212> PRT
<213> Recombinant
<400> 1

Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
                20                  25                  30
Arg Gly Asp Ala Gly Pro Lys Gly Ala Asp Gly Ala Pro Gly Ala Pro
            35                  40                  45
Gly Leu Gln Gly Met Pro Gly Glu Arg Gly Ala Ala Gly Leu Pro Gly
        50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
                100                 105                 110
Asp Ala Gly Pro Lys Gly Ala Asp Gly Ala Pro Gly Lys Asp Gly Val
            115                 120                 125
Arg Gly Leu Ala Gly Pro Ile Gly Pro Pro Gly Pro Ala Gly Ala Pro
        130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
                180                 185                 190
Gly Ala Pro Gly Leu Gln Gly Ala Pro Gly Leu Gln Gly Met Pro Gly
        195                 200                 205
Glu Arg Gly Ala Ala Gly Leu Pro Gly Pro Lys Gly Glu Arg Gly Asp
        210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
                245                 250                 255
Glu Arg Gly Asp Ala Gly Pro Lys Gly Ala Asp Gly Ala Pro Gly Lys
                260                 265                 270
Asp Gly Val Arg Gly Leu Ala Gly Pro Ile Gly Pro Pro Gly Glu Arg
                275                 280                 285
Gly Ala Ala Gly Leu Pro Gly Pro Lys Gly Glu Arg Gly Asp Ala Gly
        290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
                325                 330                 335
Gly Leu Gln Gly Met Pro Gly Glu Arg Gly Ala Ala Gly Leu Pro Gly
        340                 345                 350
Pro Lys Gly Glu Arg Gly Asp Ala Gly Pro Lys Gly Ala Asp Gly Ala
        355                 360                 365
Pro Gly Lys Asp Gly Val Arg Gly Leu Ala Gly Pro Pro Gly Ala Pro
        370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro

```
                        405                     410                       415
        Lys Gly Ala Asp Gly Ala Pro Gly Ala Pro Gly Leu Gln Gly Met Pro
                    420                     425                   430
        Gly Glu Arg Gly Ala Ala Gly Leu Pro Gly Pro Lys Gly Glu Arg Gly
                    435                     440                   445
        Asp Ala Gly Pro Lys Gly Ala Asp Gly Ala Pro Gly Lys Asp Gly Val
            450                     455                   460
        Arg Gly Leu Ala Gly Pro Ile Gly Pro Pro Gly Glu Arg Gly Ala Ala
        465                     470                   475                   480
        Gly Leu Pro Gly Pro Lys Gly Glu Arg Gly Asp Ala Gly Pro Lys Gly
                            485                     490                   495
        Ala Asp Gly Ala Pro Gly Lys Asp Gly Val Arg Gly Leu Ala Gly Pro
                        500                     505                   510
        Ile Gly Pro Pro Gly Pro Ala Gly Ala Pro Gly Ala Pro Gly Leu Gln
                    515                     520                   525
        Gly Met Pro Gly Glu Arg Gly Ala Ala Gly Leu Pro Gly Pro Lys Gly
            530                     535                   540
        Glu Arg Gly Asp Ala Gly Pro Lys Gly Ala Asp Gly Ala Pro Gly Lys
        545                     550                   555                   560
        Asp Gly Val Arg Gly Leu Ala Gly Pro Pro Gly
                            565                     570
        <210>   2
        <211>   4
        <212>   PRT
        <213>   Artificial Sequence
        <220>
        <223>   Description of Artificial Sequence: adhesive sequence
        <400>   2
        Arg Glu Asp Val
        1
        <210>   3
        <211>   5
        <212>   PRT
        <213>   Artificial Sequence
        <220>
        <223>   Description of Artificial Sequence: adhesive sequence
        <400>   3
        Tyr Ile Gly Ser Arg
        1               5
        <210>   4
        <211>   5
        <212>   PRT
        <213>   Artificial Sequence
        <220>
        <223>   Description of Artificial Sequence: adhesive sequence
        <400>   4
        Pro Asp Ser Gly Arg
        1               5
        <210>   5
        <211>   7
        <212>   PRT
        <213>   Artificial Sequence
        <220>
        <223>   Description of Artificial Sequence: adhesive sequence
        <400>   5
        Arg Tyr Val Val Leu Pro Arg
        1               5
        <210>   6
        <211>   6
        <212>   PRT
        <213>   Artificial Sequence
        <220>
        <223>   Description of Artificial Sequence: adhesive sequence
```

```
<400>   6
Leu Gly Thr Ile Pro Gly
1               5
<210>   7
<211>   10
<212>   PRT
<213>   Artificial Sequence
<220>
<223>   Description of Artificial Sequence: adhesive sequence
<400>   7
Arg Asn Ile Ala Glu Ile Ile Lys Asp Ile
1               5               10
<210>   8
<211>   5
<212>   PRT
<213>   Artificial Sequence
<220>
<223>   Description of Artificial Sequence: adhesive sequence
<400>   8
Ile Lys Val Ala Val
1               5
<210>   9
<211>   4
<212>   PRT
<213>   Artificial Sequence
<220>
<223>   Description of Artificial Sequence: adhesive sequence
<400>   9
Asp Gly Glu Ala
1
<210>   10
<211>   4
<212>   PRT
<213>   Artificial Sequence
<220>
<223>   Description of Artificial Sequence: adhesive sequence
<400>   10
Glu Arg Gly Asp
```

## Description

## BACKGROUND OF THE INVENTION

1. Field of the Invention

The present invention relates to a cell mass or cell structure-embedding agent including polypeptide in which molecular weight distribution satisfies a predetermined condition. The present invention relates to a cell mass or cell structure-containing composition and a kit including the cell mass or cell structure-embedding agent.

2. Description of the Related Art

In recent years, many regenerative medicine and cell transplantation therapies have been implemented. Particularly, treatments using cells as cell masses or cell structures have been considered. Since the cell mass or cell structure is maintained as a structure as compared with administration of individual cells as a suspension, it is considered that engraftment in the body is satisfactory and more effective.

Patent Document 1 (JP2004-357694A) discloses a method of performing osteochondral regeneration by using cell masses of tissue-derived stem cells. Patent Document 2 (WO2011/108517A) discloses a cell structure including a macromolecular block having biocompatibility and cells, in which a plurality of the macromolecular blocks are arranged in gaps between the plurality of cells. In the cell structure disclosed in WO2011/108517A, nutrient from the outside to the inside of the cell structure can be delivered, the cell has a sufficient thickness, and cells are uniformly present in the structure. In the example of Patent Document 2, high cell survival activity is demonstrated by using a macromolecular block formed of recombinant gelatin or a natural gelatin material. Patent Document 3 (JP2005-35945A) discloses a cell transplant cell structure including a macromolecular block having biocompatibility and at least one type of cells, in which a plurality of the macromolecular blocks are arranged in gaps between the plurality of cells. In the example of Patent Document 3, angiogenesis was evaluated by using a cell transplantation cell structure.

Meanwhile, Patent Document 4 (WO2008/103041A) discloses genetically modified gelatin that is particularly useful in several uses involving cell attachment, for example, cell culture work, uses involving cell culture of anchorage-dependent cells, and various medical uses.

Prior Art Documents

Patent Documents

Patent Document 1: JP2004-357694A

Patent Document 2: WO2011/108517A

Patent Document 3: JP2005-35945A

Patent Document 4: WO2008/103041A

## SUMMARY OF THE INVENTION

There are problems in that cell masses or cell structures are very fragile and are easily broken during transportation, which causes problems in a case of being transported to hospital facilities or the like. In the hospital facilities or the like, it is desirable that cell masses or cell structures can be easily transplanted after transportation of the cell masses or cell structures. It is desirable to solve the above problems in the transplantation of cell masses or cell structures. An object of the present invention is to provide a cell mass or cell structure-embedding agent that can stably transport cell masses or cell structures in a case of transportation at a low temperature and after transportation, conveniently recover a cell mass or cell structure from the cell mass or cell structure-embedding agent. Another object of the present invention is to provide a cell mass or cell structure-containing composition including the cell mass or cell structure-embedding agent and a kit including the cell mass or cell structure-embedding agent.

The present inventors have diligently conducted research to achieve the above objects and found that, according to a cell mass or cell structure-embedding agent including polypeptide which has a specific sequence and in which an area of the maximum molecular weight peak in molecular weight distribution measurement is 80% or more of the total area of all of the molecular weight peaks, it is possible to stably transport cell masses or cell structures in a case of transportation at a low temperature and after transportation, and conveniently recover a cell mass or cell structure from the cell mass or cell structure-embedding agent at room temperature in a case of transplantation of a cell mass or a cell structure. The present invention has been completed based on the finding. According to the present invention, the following inventions are provided.

[1] A cell mass or cell structure-embedding agent comprising: polypeptide which is represented by Formula 1 and in which a molecular weight distribution satisfies Condition X,

Formula 1: $A-[(Gly-X-Y)_n]_m-B$

in the formula, n X's each independently represent any amino acid, n Y's each independently represent any amino acid, m is an integer of 2 to 10, n is an integer of 3 to 100, A represents any amino acid or amino acid sequence, and B represents any amino acid or amino acid sequence,
Condition X: An area of the maximum molecular weight peak in molecular weight distribution measurement by gel permeation chromatography is 80% or more of the total area of all of the molecular weight peaks.

[2] The cell mass or cell structure-embedding agent according to [1], in which the polypeptide is recombinant gelatin.
[3] The cell mass or cell structure-embedding agent according to [1] or [2], in which the polypeptide is polypeptide represented by Formula 2,

Formula 2: $Gly-Ala-Pro-[(Gly-X-Y)_{63}]_3-Gly$

in the formula, 63 pieces of X each independently represent any amino acid and 63 pieces of Y each independently represent any amino acid, and 63 pieces of Gly-X-Y may be identical to or different from each other.
[4] The cell mass or cell structure-embedding agent according to any one of [1] to [3], in which the polypeptide has (1) an amino acid sequence presented in SEQ ID NO: 1 or (2) an amino acid sequence that has 80% or more of sequence identity with the amino acid sequence presented in SEQ ID NO: 1 and has biocompatibility.
[5] The cell mass or cell structure-embedding agent according to any one of [1] to [4], in which the polypeptide has an amino acid sequence presented in SEQ ID NO: 1.
[6] A cell mass or cell structure-containing composition comprising: a cell mass or cell structure; and the cell mass or cell structure-embedding agent according to any one of [1] to
[5], in which the cell mass or the cell structure is embedded with the cell mass or cell structure-embedding agent.
[7] A kit comprising: a cell mass or cell structure; and the cell mass or cell structure-embedding agent according to any one of [1] to [5].
[8] Polypeptide which is to be used in embedding of a cell mass or cell structure and is represented by Formula 1 and in which a molecular weight distribution satisfies Condition X,

Formula 1: $A-[(Gly-X-Y)_n]_m-B$

in the formula, n X's each independently represent any one of amino acids, n Y's each independently represent any amino acids, m is an integer of 2 to 10, n is an integer of 3 to 100, A represents any amino acid or amino acid sequence, and B represents any amino acid or amino acid sequence,
Condition X: An area of the maximum molecular weight peak in molecular weight distribution measurement by gel permeation chromatography is 80% or more of the total area of all of the molecular weight peaks.

[9] A use of polypeptide which is to be used in manufacturing of a cell mass or cell structure-embedding agent and is represented by Formula 1 and in which a molecular weight distribution satisfies Condition X,

Formula 1: $A-[(Gly-X-Y)_n]_m-B$

in the formula, n X's each independently represent any one of amino acids, n Y's each independently represent any amino acids, m is an integer of 2 to 10, n is an integer of 3 to 100, A represents any amino acid or amino acid sequence, and B represents any amino acid or amino acid sequence,
Condition X: An area of the maximum molecular weight peak in molecular weight distribution measurement by gel permeation chromatography is 80% or more of the total area of all of the molecular weight peaks.

[10] A method of embedding a cell mass or cell structure, the method including: embedding a cell mass or cell structure with polypeptide which is represented by Formula 1 and in which a molecular weight distribution satisfies Condition X,

Formula 1: $A-[(Gly-X-Y)_n]_m-B$

in the formula, n X's each independently represent any one of amino acids, n Y's each independently represent any amino acids, m is an integer of 2 to 10, n is an integer of 3 to 100, A represents any amino acid or amino acid sequence, and B represents any amino acid or amino acid sequence,
Condition X: An area of the maximum molecular weight peak in molecular weight distribution measurement by gel permeation chromatography is 80% or more of the total area of all of the molecular weight peaks.

According to the cell mass or cell structure-embedding agent, the cell mass or cell structure-containing composition, and the kit according to the embodiment of the present invention, a cell mass or cell structure can be stably transported in a case of low temperature transportation, and after the transportation, a cell mass or a cell structure can be easily recovered from cell mass or cell structure-embedding agent.

## BRIEF DESCRIPTION OF THE DRAWINGS

Fig. 1 illustrates molecular weight distribution of recombinant gelatin.
Fig. 2 illustrates molecular weight distribution of natural animal gelatin.
Fig. 3 illustrates shapes of CBE3 embedded cell structures before and after shaking.

## DESCRIPTION OF THE PREFERRED EMBODIMENTS

Hereinafter, an embodiment of the present invention will be described in detail.

<Cell mass or cell structure-embedding agent>

The cell mass or cell structure-embedding agent according to the embodiment of the present invention is a cell mass or cell structure-embedding agent including polypeptide which is represented by Formula 1 and in which molecular weight distribution satisfies Condition X.

Formula 1: $A-[(Gly-X-Y)_n]_m-B$

In the formula, n X's each independently represent any amino acids, n Y's each independently represent any amino acids, m is an integer of 2 to 10, n is an integer of 3 to 100, A represents any amino acid or amino acid sequence, and B represents any amino acid or amino acid sequence.
Condition X: An area of the maximum molecular weight peak in molecular weight distribution measurement by gel permeation chromatography is 80% or more of the total area of the all molecular weight peaks.
The cell mass or cell structure-embedding agent described in the present specification is a material that can perform an embedding treatment on a cell mass or a cell structure. The embedding treatment is a treatment of causing a portion or an entire portion of the surface of the cell mass or the cell structure to come into contact with the cell mass or cell structure-embedding agent by immersion or the like and covering the portion or the entire portion of the surface of the cell mass or the cell structure with the cell mass or cell structure-embedding agent.

[Polypeptide]

The polypeptide represented by Formula 1 is gelled by embedding cell masses or cell structures in a solution state (not a low temperature condition) and reducing the temperature, to be in a state that is suitable for transportation. In view of cell preservation, it is necessary to keep the temperature low during the transportation. In the present invention, polypeptide is sharply gelled at a low temperature (4°C) and the dissolution at room temperature (25°C) by satisfying Condition X (the area of the maximum molecular weight peak in the molecular weight distribution measurement by gel permeation chromatography is 80% or more of the total area of the all molecular weight peaks), and in a case of transplantation of a cell mass or a cell structure, a cell mass or a cell structure can be easily recovered from the cell mass or cell structure-embedding agent at room temperature.

As described above, in the present invention, the area of the maximum molecular weight peak in the molecular weight distribution measurement by the gel permeation chromatography is 80% or more, more preferably 85% or more, and particularly preferably 90% or more of the total area of the all molecular weight peaks.

The molecular weight distribution by gel permeation chromatography can be measured by using high performance liquid chromatography (HPLC) (AQUITY UPLC system Empower 2 manufactured by Waters Corporation) and using 100 mmol/L of a phosphate buffer (pH 6.8) as a buffer solution.

In Formula 1, m is an integer of 2 to 10 and preferably 3 to 5.

In Formula 1, n is an integer of 3 to 100, preferably an integer of 15 to 70, and more preferably an integer of 50 to 65.

The polypeptide represented by Formula 1 used in the present invention may be any of recombinant polypeptide, chemically synthesized polypeptide, or natural polypeptide, as long as the molecular weight distribution satisfies the above condition X.

Chemically synthesized polypeptide means artificially synthesized polypeptide. The synthesis of a polypeptide may be solid phase synthesis or liquid phase synthesis, but is preferably solid phase synthesis. The solid phase synthesis of a polypeptide is well-known to those skilled in the art, and examples thereof include a fluorenyl-methoxy-carbonyl group (Fmoc group) synthesis method in which a Fmoc group is used for protection of an amino group, and a tert-butyl oxy carbonyl group (Boc group) synthesis method in which a Boc group is used for protection of an amino group.

The polypeptide is preferably a recombinant polypeptide. In the present specification, recombinant polypeptide represented by Formula 1 is referred to as recombinant gelatin. The recombinant gelatin will be described below in the present specification.

A "1/IOB" value which is a hydrophilicity value of the polypeptide used in the present invention is preferably 0 to 1.0. The value is more preferably within a range of 0 to 0.6, and even more preferably within a range of 0 to 0.4. IOB is an index of hydrophilic and hydrophobic properties based on an organic conceptual diagram representing polarity and non-polarity of an organic compound proposed by Atsushi HUJITA, and the details thereof are described in, for example, "Pharmaceutical Bulletin", vol. 2, 2, pp. 163 to 173 (1954), "Area of Chemistry" vol. 11, 10, pp. 719-725 (1957), and "Fragrance Journal, vol. 50, pp. 79 to 82 (1981). Briefly, the root of every organic compound is set to methane ($CH_4$), and all of other compounds are regarded as derivatives of methane. Certain numerical values for the number of carbons thereof, a substituent group, a transformation portion, a ring, and the like are set, and an organic value (OV) and an inorganic value (IV) are obtained by adding the score thereof. These values are plotted on a diagram in which the organic value is represented on the X-axis and the inorganic value is represented on the Y-axis. IOB in the organic conceptual diagram refers to a ratio of the inorganic value (IV) to the organic value (OV) in the organic conceptual diagram, that is, "inorganic value (IV)/organic value (OV)". The details of the organic conceptual diagram can be referred to "New Edition Organic Conceptual Diagram -Foundation and Application-" (written by Yoshio KOUDA, Sankyo Shuppan Co., Ltd., 2008). In the present specification, the hydrophilic and hydrophobic properties are represented by a "1/IOB" value which was obtained by taking a reciprocal number of IOB. This is a notation of representing more hydrophilic properties as the "1/IOB" value becomes small (close to 0).

The hydrophilic properties and water absorbency are increased by causing the "1/IOB" value of the polypeptide used in the present invention to be within the above-described range.

With respect to the polypeptide used in the present invention, the hydrophilic and hydrophobic indexes represented by a grand average of hydropathicity (GRAVY) value are preferably -9.0 to 0.3, and more preferably -7.0 to 0.0. The grand average of hydropathicity (GRAVY) value can be obtained by methods of "Gasteiger E., Hoogland C., Gattiker A., Duvaud S., Wilkins M.R., Appel R.D., Bairoch A.; Protein Identification and Analysis Tools on the ExPASy Server; (In) John M. Walker (ed): The Proteomics Protocols Handbook, Humana Press (2005). pp. 571 to 607" and "Gasteiger E., Gattiker A., Hoogland C., Ivanyi I., Appel R.D., Bairoch A.; ExPASy: the proteomics server for in-depth protein knowledge and analysis.; Nucleic Acids Res. 31:3784 to 3788 (2003)".

The hydrophilic properties and water absorbency become high by making the GRAVY value of the polypeptide used in the present invention be within the above-described range.

[Recombinant gelatin]

The polypeptide used in the present invention is preferably recombinant gelatin.

Examples thereof include recombinant gelatin disclosed in EP1014176, US6992172B, WO2004/85473A, and WO2008/103041A, but the recombinant gelatin is not limited thereto. Preferred recombinant gelatin used in the present invention is recombinant gelatin of the following aspect.

The recombinant gelatin is excellent in biocompatibility with original performance of natural gelatin, and is excellent in non-infection properties since there is no concern of bovine spongiform encephalopathy (BSE) and the recombinant gelatin with not being naturally derived.

The molecular weight of recombinant gelatin is not particularly limited, but is preferably 2,000 to 100,000 (2 kDa (kilodaltons) to 100 kDa), more preferably (2,500 to 95,000 (2.5 kDa to 95 kDa), even more preferably 5,000 to 90,000 (5 kDa to 90 kDa), and most preferably 10,000 to 90,000 (10 kDa to 90 kDa).

The recombinant gelatin preferably has a repetition of a sequence represented by Gly-X-Y which is characteristic to collagen. Here, a plurality of pieces of Gly-X-Y may be identical to or different from each other. In Gly-X-Y, Gly represents glycine and X and Y represent any amino acid (preferably represents any amino acid other than glycine). The sequence represented by Gly-X-Y characteristic to collagen is a partial structure which is extremely specific compared to other protein in a composition or a sequence of an amino acid of gelatin/collagen. In this section, glycine occupies about one third of the entirety of the amino acid sequence, one sequence is repeated every three sequences. Glycine is the simplest amino acid. Therefore, there is a little restraint in arrangement of molecular chains and glycine significantly contributes to regeneration of a helix structure during gelation. It is preferable that amino acids represented by X and Y contain many imino acids (proline and oxyproline) and occupy 10% to 45% of the entirety of the sequence. Preferably 80% or more of the sequence of the amino acids, more preferably 95% or more of the sequence of the amino acids, and most preferably 99% or more of the sequence of the amino acids in the recombinant gelatin have a repeating structure of Gly-X-Y.

In general gelatin, a polar amino acid with an electrical charge and a polar non-charged amino acid exist by 1:1 in polar amino acids. Here, the polar amino acid specifically indicates cysteine, aspartic acid, glutamic acid, histidine, lysine, asparagine, glutamine, serine, threonine, tyrosine, or arginine. Among these, the polar non-charged amino acid indicates cysteine, asparagine, glutamine, serine, threonine, or tyrosine. In recombinant gelatin used in the present invention, the proportion of the polar amino acid in the whole constituent amino acid is 10% to 40% and preferably 20% to 30%. The proportion of a non-charged amino acid in the polar amino acid is preferably greater than or equal to 5% and less than 20% and more preferably greater than or equal to 5% and less than 10%. It is preferable that any one amino acid or preferably two or more amino acids among serine, threonine, asparagine, tyrosine, and cysteine are not contained on a sequence.

In general, in polypeptides, minimum amino acid sequences which work as cell adhesion signals are known (for example, Nagai Shoten Co., Ltd., "Pathophysiology", Vol. 9, No. 7 (1990) p. 527). The recombinant gelatin used in the present invention preferably has two or more of these cell adhesion signals in one molecule. As the specific sequences, sequences such as an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence, which are represented by one-letter notation of amino acids are preferable in that there are many kinds of cells adhered. An RGD sequence, a YIGSR sequence, a PDSGR sequence, an LGTIPG sequence, an IKVAV sequence, and a HAV sequence are more preferable and an RGD sequence is particularly preferable. In the RGD sequence, an ERGD sequence is preferable.

As arrangement of RGD sequences in recombinant gelatin used in the present invention, it is preferable that the number of amino acids between RGDs is between 0 to 100 and preferably between 25 to 60 without being even.

The content of this minimum amino acid sequence is preferably 3 to 50, more preferably 4 to 30, and particularly preferably 5 to 20 in one molecule of protein. The most preferable content thereof is 12.

In recombinant gelatin used in the present invention, the proportion of RGD motifs with respect to the total number of amino acids is preferably at least 0.4%. In a case where recombinant gelatin contains 350 or more amino acids, each stretch of the 350 amino acids preferably contains at least one RGD motif. The proportion of RGD motifs is more preferably at least 0.6%, even more preferably at least 0.8%, still even more preferably at least 1.0%, particularly preferably at least 1.2%, and most preferably at least 1.5% with respect to the total number of amino acids. The number of RGD motifs within a recombinant peptide is preferably at least 4, more preferably 6, even more preferably 8, and particularly preferably 12 to 16 per 250 amino acids. The proportion of RGD motifs being 0.4% corresponds to at least one RGD sequence per 250 amino acids. The number of RGD motifs is an integer, and therefore, gelatin formed of 251 amino acids needs to contain at least two RGD sequences in order to satisfy the characteristics of 0.4%. It is preferable that the recombinant gelatin of the present invention contains at least two RGD sequences per 250 amino acids, more preferably contains at least three RGD sequences per 250 amino acids, and even more preferably contains at least four RGD sequences per 250 amino acids. As a further mode of the recombinant gelatin of the present invention, the recombinant gelatin contains at least 4 RGD motifs, preferably 6 RGD motifs, more preferably 8 RGD motifs, and even more preferably 12 to 16 RGD motifs.

In addition, the recombinant gelatin may be partially hydrolyzed.

The polypeptide used in the present invention is more preferably represented by Formula 2.

Formula (2):  Gly-Ala-Pro-[(Gly-X-Y)$_{63}$]$_3$-Gly

in the formula, 63 pieces of X each independently represent any amino acid and 63 pieces of Y each independently represent any amino acid, and 63 pieces of Gly-X-Y may be identical to or different from each other.

It is preferable that a plurality of sequence units of collagen which naturally exists are bonded to a repeating unit. Any naturally existing collagen referred to herein may be used as long as the collagen naturally exists, but is preferably I type collagen, II type collagen, III type collagen, IV type collagen, or V type collagen, and more preferably I type collagen, II type collagen, or III type collagen. According to another form, the above-described collagen is preferably derived from a human-type, cattle, a pig, a mouse, or a rat, and is more preferably derived from a human-type.

An isoelectric point of the recombinant gelatin used in the present invention is preferably 5 to 10, more preferably 6 to 10, and even more preferably 7 to 9.5. The measurement of the isoelectric point of the recombinant gelatin can be carried out by measuring the pH after passing a 1 mass% gelatin solution through a mixed crystal column of a cation-anion exchange resin above-described disclosed in isoelectric focusing method (refer to Maxey, C. R. (1976; Phitogr. Gelatin 2, Editor Cox, P. J. Academic, London, Engl.)).

It is preferable that the recombinant gelatin is not deaminated.

It is preferable that the recombinant gelatin does not have a telopeptide.

It is preferable that the recombinant gelatin is a substantially pure polypeptide which is prepared using a nucleic acid encoding an amino acid sequence.

The recombinant gelatin is particularly preferably

(1) an amino acid sequence described in SEQ ID No: 1; or
(2) an amino acid sequence having 80% or more (preferably 90% or more, more preferably 95% or more, and particularly preferably 98% or more) sequence identity to the amino acid sequence described in SEQ ID No: 1 and has biocompatibility.

Biocompatibility means that, in a case of being brought into contact with a living body, it does not give a rise to a remarkable adverse reaction such as long-term and chronic inflammatory reaction.

The recombinant gelatin most preferably has the amino acid sequence described in SEQ ID No: 1.

The sequence identity of the embodiment of the present invention refers to a value calculated in the following equation.

$$\% \text{ Sequence identity} = [(\text{the number of identical residues}) / (\text{alignment length})] \times 100$$

The sequence identity between two amino acid sequences can be determined by any method well-known to those skilled in the art and can be determined by the Basic Local Alignment Search Tool (BLAST) program (J. Mol. Biol. 215: 403 to 410, 1990) or the like.

The recombinant gelatin is formed of an amino acid sequence in which one or several amino acids are deleted, substituted, or added in the amino acid sequence described in SEQ ID No: 1 and has biocompatibility.

"One or several" in the expression "amino acid sequence in which one or several amino acids are deleted, substituted, or added" preferably means 1 to 20 amino acids, more preferably means 1 to 10 amino acids, even more preferably means 1 to 5 amino acids, and particularly preferably means 1 to 3 amino acids.

The recombinant gelatin can be manufactured through gene recombination technology which is known to those skilled in the art, and can be manufactured in accordance with, for example, methods disclosed in EP1014176A2, US6992172B, WO2004/85473A, and WO2008/103041A. Specifically, a gene encoding an amino acid sequence of predetermined recombinant gelatin is acquired, the acquired gene is incorporated into an expression vector to manufacture a recombinant expression vector, and a transformant is manufactured by introducing the recombinant expression vector into an appropriate host. The recombinant gelatin is produced by culturing the obtained transformant in an appropriate medium. Therefore, it is possible to prepare the recombinant gelatin used in the present invention by collecting the recombinant gelatin produced from a culture product.

[Form of cell mass or cell structure-embedding agent]

The cell mass or cell structure-embedding agent according to the embodiment of the present invention is not particularly limited, and can have any forms, as long as the cell mass or cell structure-embedding agent includes polypeptide that is represented by Formula 1 and of which the molecular weight distribution satisfies Condition X. For example, the form may be a solution (such as an aqueous solution), a suspension, a powder, or a gel. In the case of powder or gel, the cell mass or cell structure-embedding agent can be used by dissolving in a solvent such as water in a case use.

The content of the polypeptide in the cell mass or cell structure-embedding agent according to the embodiment of the present invention is not particularly limited, and is generally 0.1 mass% to 100 mass% and preferably 0.5 mass% to 100 mass%.

The cell mass or the cell structure to which the cell mass or cell structure-embedding agent according to the embodiment of the present invention is applied is described below in the present specification.

<Cell mass or cell structure-containing composition>

The present invention relates to a cell mass or cell structure-containing composition including a cell mass or a cell structure, and the cell mass or cell structure-embedding agent according to the embodiment of the present invention, and the cell mass or the cell structure is embedded by the cell mass or the cell structure-embedding agent.

[Cell mass]

The cell mass of the present invention is in a state in which a plurality of cells are associated into one mass and is a cell mass a diameter of one mass is 100 $\mu$m or more, and a value of a major axis / a minor axis of one mass is 200 or less.

The cells may be linked to each other directly and / or via an inclusion. The inclusion is not particularly limited as long as the inclusion is a material capable of at least mechanically linking cells, and examples thereof include an extracellular matrix. The inclusion is preferably a cell-derived material, particularly, a material derived from a cell constituting a cell mass or a cell structure. The cells are at least mechanically linked, but may be further functionally, for example, chemically and electrically linked to each other.

The cell mass can be produced by a well-known cell mass manufacturing method or an equivalent method thereto. For example, cells may be cultured in a U-shaped bottom plate or a multiwell dish, and after the cells are aggregated, the mass may be recovered from the culture dish. As another method, cell clumps can be manufactured by self-aggregation of cells by stirring the cells. As described above, the method for manufacturing a cell mass is not particularly limited, but as an example, a cell mass or a cell structure can be manufactured by a method disclosed in JP1993-268933A (JP-H05-268933A).

A cell mass is typically manufactured by a step of seeding cells in a culture dish and a step of culturing the cells to form a cell mass. As another method, a cell mass is manufactured by a step of culturing the cells with stirring.

The cells can be cultured under conditions commonly used in the art. For example, typical culture conditions include culturing a cell at 37°C in 5%$CO_2$.

[Cell Structure]

A cell structure in the present invention means that cells and cell supports are in contact with each other or are adhered to each other to have a three-dimensional form.

As the cell structure, a cell structure that includes a biocompatible macromolecular block and a cell and in which the plurality of biocompatible macromolecular blocks are disposed in gaps between the plurality of cells is preferable.

[Biocompatible macromolecular block]

(1) Biocompatible macromolecule

Biocompatibility means that, in a case of being brought into contact with a living body, it does not give a rise to a remarkable adverse reaction such as long-term and chronic inflammatory reaction. Whether or not the biocompatible macromolecules used in the present invention are decomposed within a living body is not particularly limited as long as the biocompatible macromolecules have affinity to the living body. However, biodegradable macromolecules are preferable. Specific examples of non-biodegradable materials include polytetrafluoroethylene (PTFE), polyurethane, polypropylene, polyester, vinyl chloride, polycarbonate, acryl, stainless steel, titanium, silicone, and 2-methacryloyloxyethyl phosphorylcholine (MPC). Specific examples of the biodegradable materials include naturally derived peptides, polypeptides such as a recombinant peptide or a chemically synthesized peptide, polylactic acid, polyglycolic acid, lactic acid-glycolic acid copolymers (PLGA), hyaluronic acid, glycosaminoglycan, proteoglycan, chondroitin, cellulose, agarose, carboxymethyl cellulose, chitin, and chitosan. Among the above, polypeptide is particularly preferable. Devising of an improvement of cell adhesion properties in these biocompatible macromolecules may be performed. Specifically, methods of "coating with the cell adhering substrate (fibronectin, vitronectin, laminin) and the cell adhesion sequence (RGD sequence, LDV sequence, REDV sequence, YIGSR sequence, PDSGR sequence, RYVVLPR sequence, LGTIPG sequence, RNIAEIIKDI sequence, IKVAV sequence, LRE sequence, DGEA sequence, and HAV sequence) peptide", "amination and cationization of the substrate surface", or "plasma treatment of the substrate surface" can be used.

Polypeptide suitable as a biocompatibility polymer is the same as the polypeptide represented by Formula 1, which is used in the present invention.

(2) Cross-linking

The biocompatible macromolecules may be or may not be cross-linked, but are preferably cross-linked. By using the cross-linked biocompatible macromolecules, it is possible to obtain an effect of preventing instant decomposition during culturing in a medium and during transplantation into a living body. As general cross-linking methods, thermal cross-linking, cross-linking using aldehydes (for example, formaldehyde or glutaraldehyde), cross-linking using a condensation agent (carbodiimide, cyanamide, or the like), enzymatic cross-linking, photocrosslinking, ultraviolet cross-linking, a hydrophobic interaction, hydrogen bonding, an ionic interaction, and the like are known, it is also possible to use the above-described cross-linking methods of the embodiment of the present invention. As the cross-linking methods used in the present invention, thermal cross-linking, ultraviolet cross-linking, or enzymatic cross-linking is more preferable, and thermal cross-linking is particularly preferable.

In a case of performing cross-linking using an enzyme, there is no particular limitation as long as the enzyme has a cross-linking action between macromolecular materials. However, it is possible to perform cross-linking preferably using transglutaminase and laccase and most preferably using transglutaminase. Specific examples of protein to be subjected to enzymatic cross-linking using transglutaminase are not particularly limited as long as the protein has a lysine residue and a glutamine residue. Transglutaminase may be derived from a mammal or may be derived from a microorganism. Specific examples thereof include mammal derived transglutaminase which has been sold as Activa series manufactured by Ajinomoto Co., Inc., and a reagent; guinea pig liver derived transglutaminase manufactured by, for example, Oriental Yeast Co., Ltd., Upstate USA Inc., or Biodesign International, Inc.; goat derived transglutaminase; rabbit derived transglutaminase; and human derived blood coagulation factors (Factor XIIIa: Haematologic Technologies, Inc).

The reaction temperature in a case of performing cross-linking (for example, thermal cross-linking) is not particularly limited as long as cross-linking can be performed, but is preferably -100°C to 500°C, more preferably 0°C to 300°C, even more preferably 50°C to 300°C, particularly preferably 100°C to 250°C, and most preferably 120°C to 200°C.

(3) Biocompatible Macromolecular Block

The shape of the biocompatible macromolecular block is not particularly limited. Examples thereof include an amorphous shape, a spherical shape, a particulate shape (granule), a powdery shape, a porous shape, a fibrous shape, a spindle shape, a flat shape, and a sheet shape. An amorphous shape, a spherical shape, a particulate shape (granule), a powdery shape, and a porous shape are preferable. The amorphous shape indicates that the shape of a surface is uneven, and indicates, for example, an object, such as rock, which has roughness. Examples of the above-described shapes are not distinct from each other. For example, in some cases, an example of a subordinate concept of the particulate shape (granule) is an amorphous shape.

The size of one biocompatible macromolecular block is preferably 1 μm to 700 μm, more preferably 10 μm to 700 μm, even more preferably 10 μm to 300 μm, and still even more preferably 20 μm to 150 μm.

The method for producing a biocompatible macromolecular block is not particularly limited. For example, it is possible to obtain a biocompatible macromolecular block by pulverizing a solid matter (such as a porous body of a biocompatible macromolecule) containing a biocompatible macromolecule using a pulverizer (such as NEW POWERMILL). The solid matter (such as a porous body of a biocompatible macromolecule) containing a biocompatible macromolecule can be obtained, for example, by freeze-drying an aqueous solution containing the biocompatible macromolecule.

[Cell]

The cell mass or cell structure or the cell structure according to the embodiment of the present invention includes any cells that can form a cell mass or cell structure or a cell structure. Cells to be used are preferably animal cells, more preferably vertebrate derived cells, and particularly preferably human derived cells. The types of vertebrate derived cells (particularly, human-type derived cells) may be any of universal cells, somatic stem cells, precursor cells, and mature cells and particularly preferably somatic stem cells.

It is possible to use, for example, embryonic stem (ES) cells, germ-stem (GS) cells, or artificial pluripotent stem (iPS) cells as the universal cells. It is possible to use, for example, mesenchymal stem cells (MSC), hematopoietic stem cells, amniotic cells, umbilical cord blood cells, bone marrow derived cells (for example, bone marrow derived MSCs), myocardial stem cells, adipose derived stem cells, or neural stem cells can be used as the somatic stem cell. It is possible to use, for example, skin, dermis, epidermis, muscle, cardiac muscles, nerves, bones, cartilage, endothelium, brain, epithelium, heart, kidney, liver, pancreas, spleen, oral cavity, cornea, bone marrow, umbilical cord blood, amnion, or cells derived from hair as the precursor cells and the mature cells. It is possible to use, for example, ES cells, iPS cells, MSCs, chondrocytes, osteoblasts, osteoprecursor cells, mesenchymal cells, myoblasts, cardiac muscle cells, cardiomyoblasts, nerve cells, hepatocytes, beta cells, fibroblasts, corneal endothelial cells, vascular endothelial cells, corneal epithelial cells, amniotic cells, umbilical cord blood cells, bone marrow-derived cells, or hematopoietic stem cells as the human-type-derived cells. In addition, the cells may be derived from any of autologous cells and heterologous cells.

[Cell Structure]

The cell structure in the present invention is a cell structure in which the plurality of macromolecular blocks having biocompatibility and the cell are used, and the plurality of macromolecular blocks are three-dimensionally arranged in gaps between a plurality of cells in a mosaic shape.

The thickness or the diameter of the cell structure can be caused to be a desired thickness, but the lower limit is preferably 150 μm or more, more preferably 215 μm or more, and most preferably 400 μm or more. The upper limit of the thickness or the diameter is not particularly limited, but the general range in use is preferably 3 cm or less, more preferably 2 cm or less, and even more preferably 1 cm or less.

The cell structure can be manufactured by alternately arranging biocompatible macromolecular blocks and cells. The manufacturing method is not particularly limited, but is preferably a method of seeding cells after a biocompatible macromolecular block is formed. Specifically, cell structures can be manufactured by incubating a mixture of the biocompatible macromolecular blocks and a cell-containing culture solution. For example, the cells and the macromolecular blocks having biocompatibility manufactured in advance are arranged in a mosaic shape in a container or in a solution held in a container. As means of disposition, it is preferable to promote and control mosaic-like disposition consisting of the cells and the biocompatible substrate by using natural aggregation, natural falling, centrifugation, and stirring.

The container to be used is preferably a container consisting of a cell low adhesive material or a cell non-adhesive material and more preferably a container consisting of polystyrene, polypropylene, polyethylene, glass, polycarbonate, and polyethylene terephthalate. The shape of the bottom surface of the container is preferably a flat bottom shape, a U shape, or a V shape. As the container, a multiwell-type container may be used.

[Manufacturing of cell mass or cell structure-containing composition]

A cell mass or cell structure-containing composition can be manufactured by embedding the cell mass or cell structure by the cell mass or cell structure-embedding agent according to the embodiment of the present invention. The embedding method is not particularly limited, but the embedding agent (preferably solution) of the present invention may be added to a container including a cell mass or a cell structure and cooled at a low temperature (for example, 2°C to 12°C) to gell the solution. According to the above, it is possible to manufacture a cell mass or cell structure-containing composition in which a cell mass or cell structure is embedded with a cell mass or cell structure-embedding agent.

<Kit>

According to the present invention, there is provided a kit including the cell mass or cell structure and the cell mass or cell structure-embedding agent according to the embodiment of the present invention. Details and preferable aspects of the cell mass or cell structure and the cell mass or cell structure-embedding agent are as described above in the present specification. The kit may further include a container for performing an embedding treatment, an instruction manual, and the like.

The present invention will be more specifically described using the following examples, but is not limited by the examples.

Examples

(1) Recombinant Gelatin

The following CBE3 (which is disclosed in WO2008/103041A) was prepared as recombinant gelatin.

CBE3:

Molecular weight: 51.6 kD

Structure: GAP[(GXY)_{63}]_3G
Number of amino acids: 571
RGD sequence: 12
Imino acid content: 33%

Almost 100% of amino acids have a repeating structure of GXY. In the amino acid sequence of CBE3, serine, threonine, asparagine, tyrosine, and cysteine are not included. CBE3 has an ERGD sequence.
Isoelectric point: 9.34
GRAVY value: -0.682
1/IOB value: 0.323

Amino acid sequence (SEQ ID No: 1 in a sequence table) (which is the same as that of SEQ ID No: 3 in WO2008/103041A. However, X in the end is corrected to "P").

GAP(GAPGLQGAPGLQGMPGERGAAGLPGPKGERGDAGPKGADGAPGAPGL

QGMPGERGAAGLPGPKGERGDAGPKGADGAPGKDGVRGLAGPIGPPGERGAAGLPG

PKGERGDAGPKGADGAPGKDGVRGLAGPIGPPGPAGAPGAPGLQGMPGERGAAGLP

GPKGERGDAGPKGADGAPGKDGVRGLAGPP)3G

(2) Measurement of molecular weight distribution of recombinant gelatin

With respect to the recombinant gelatin CBE3 (Example 1) described in (1), molecular weight distribution was measured by using gel permeation chromatography (GPC). In the measurement of the molecular weight distribution, HPLC (AQUITY UPLC system Empower 2 manufactured by Waters Corporation) was used, and 100 mmol/L of a phosphate buffer (pH 6.8) was used as a buffer solution. Relativity of the molecular weight distribution of recombinant gelatin was measured by using these.

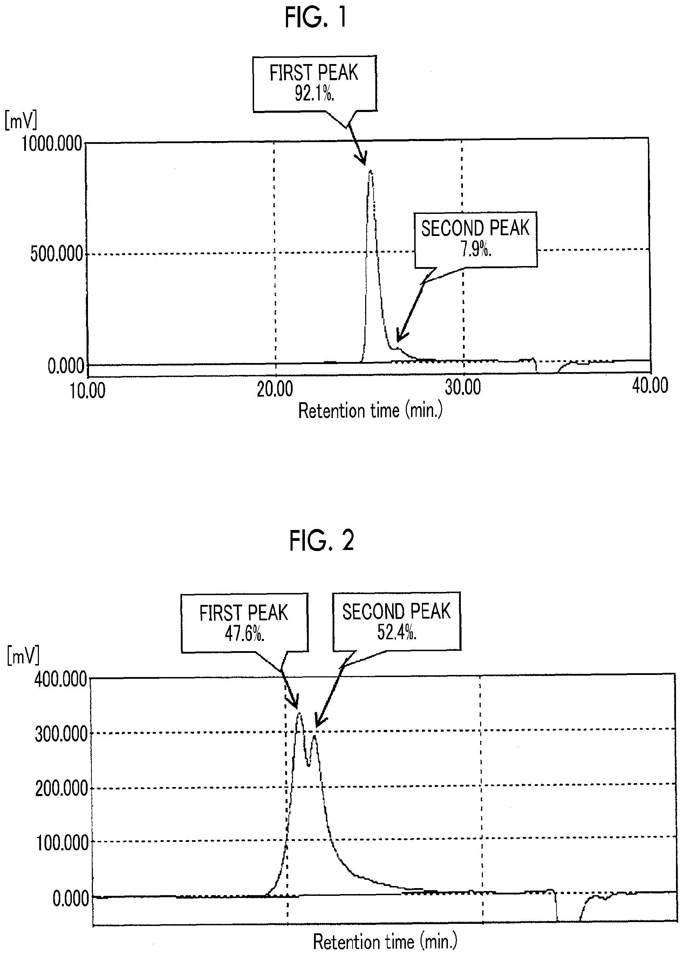

The results thereof are provided in Fig. 1. Two molecular weight peaks of CBE3 were present. In a case where occupancies of the peaks in all the peaks were calculated from the area ratio for these two peaks, the occupancy that of the first peak was 92.1% and the occupancy of the second peak was 7.9%. From this, it was found that CBE3 had a molecular weight peak that occupies 92% as a high occupancy peak.

(3) Measurement of molecular weight distribution of natural animal gelatin

In comparison, for natural animal gelatin (Comparative Example 1), the molecular weight distribution was measured by using GPC in the same manner as in (2) above. The results are provided in Fig. 2. Two molecular weight peaks of the natural animal gelatin were present. In a case where occupancies of the peaks in all the peaks were calculated from the area ratio for these two peaks, the occupancy that of the first peak was 47.6%, and the occupancy of the second peak was 52.4%. From this, it was found that the natural animal gelatin had a molecular weight peak that occupies 52% as a high occupancy peak.

(4) Embedding agent performance test (gelation rate)

In order to use as an embedding agent in a case of transporting a cell mass or a cell structure, the performance in which the gelation rate at low temperature is fast, and conversely, the embedding agent quickly returns to liquid at room temperature, as a reversible reaction becomes important. Therefore, the gelation rate at low temperature and the speed of returning to the liquid at room temperature were tested at a concentration of 1 mass%.

As a result, it was observed that CBE3 of Example 1 was rapidly gelled by returning to 4°C, and the fluidity of the liquid rapidly decreased, and in a case where the completely gelled sample was transferred to a room temperature environment of 25°C, CBE3 quickly returns to the liquid.

Specifically, in CBE3, gelation started after 40 minutes from returning to 4°C, and almost stable gel was obtained after 50 minutes. The gel was a strong gel which was not crushed even in a case of being pressed by hand. The gel returned to room temperature at 25°C and returned to a complete liquid in 40 minutes.

Meanwhile, the natural animal gelatin of Comparative Example 1 was gelled by returning to 4°C, but the speed thereof was gradual and slower than that of CBE3. As a result of transferring the completely gelled sample to a room temperature environment of 25°C and testing the speed of returning to the liquid again, in the natural animal gelatin of Comparative Example 1, the speed of returning to liquid was slower than that in CBE3 of Example 1.

Specifically, in the natural animal gelatin, the gelation started after 60 minutes by transferring to 4°C, but only a loose gel was obtained even after 90 minutes. After that, the natural animal gelatin did not transfer to a strong gel, and remained in a loose gel state that was able to be easily crushed by hand pressing. In a case where the gell-state material was returned to room temperature of 25°C, it took 70 minutes for the natural animal gelatin to return to a complete liquid.

(5) Manufacturing of CBE3 embedded cell mass or CBE3 embedded cell structure

Human bone marrow-derived mesenchymal stem cells (hMSCs) were suspended in a proliferation medium (Takara Bio Inc.: MSCGM Bullet Kit (trademark)), biocompatible macromolecular blocks (53 to 106 $\mu$m) as in WO2015046216A1 were added thereto, in a state in which hMSCs ($1.2 \times 10^6$ cells) and biocompatible macromolecular blocks (1 mg) were finally suspended in 4 mL of a medium, the mixture was sown in EZSPHERE (registered trademark) DISH Type 903 (which had a spheroid well diameter of 800 $\mu$m, a spheroid well depth of 300 $\mu$m, and about 1,000 spheroid wells, in which bottom surface was a culture surface having a recess portion, and has a side outer wall erected on the periphery of the culture surface, and which was manufactured by AGC TECHNO GLASS CO., Ltd.) which was a cell non-adhesive 35 mm dish. After culturing for 69 hours, about 1,000 cell structures were obtained. A cell mass was also able to be obtained by carrying out the same operation without adding a biocompatible macromolecular block.

The obtained cell structures or cell masses were separated into several cell structures or cell masses in 96 well plates, 200 uL of HBSS + (manufactured by Thermo Fisher Scientific) including 1 mass% of CBE3 was added, and cooling was performed at 2°C to 8°C for one hour for gelation, so as to manufacture a CBE3 embedded cell mass or a CBE3 embedded cell structure. HBSS is an abbreviation of Hanks' Balanced Salt solution.

(6) Vibration evaluation

(6-1)

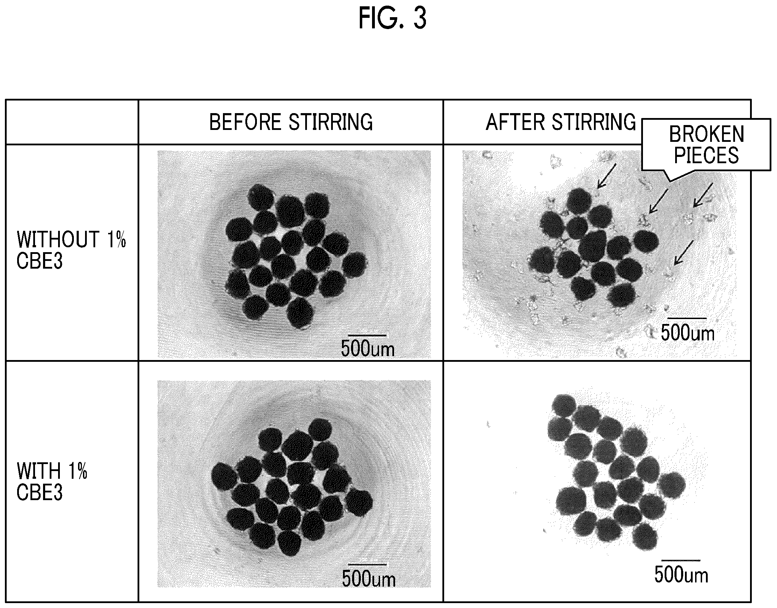

The CBE3 embedded cell mass or the CBE3 embedded cell structure manufactured in (5) above was placed on a MicroPlateMixer (NS-P, manufactured by As One Corporation), and was shaken at 2°C to 8°C for two days with the maximum number of shaking set. As a control, HBSS+ without CBE3 was added to the cell mass or cell structure, and shaken in the same manner. After the shaking, the CBE3 embedded cell mass or the CBE3 embedded cell structure was left at room temperature, and the gel was thawed to recover the cell mass or cell structure. The shape of the cell structure before and after the shaking is shown in Fig. 3.

In a case where HBSS+ without CBE3 was added, the cell mass or cell structure after the shaking was broken, but, in a case where the cell mass or cell structure was embedded with HBSS+ including 1 mass% of CBE3, the cell mass or cell structure was not broken even after the shaking.

(6-2)

In a case where HBSS+ including 1 mass% of CBE3 was added, the cell mass or cell structure recovered after shaking was cultured, so as to check that the cell mass or cell structures were fused with each other. This indicates that the cells in the cell mass or cell structure are alive, and it was checked that the cell mass or cell structure can sufficiently exhibit the performance thereof.

[SEQUENCE LISTING] International Application 17F02612 cell mass or cell structure embedding agent JP18007943   20180302----00030043851800434061   Normal   201803021059022018021417155955530_P1A-P101_17_0.app based on the International Patent Cooperation Treaty

**Claims**

1. A cell mass or cell structure-embedding agent comprising: polypeptide which is represented by Formula 1 and in which a molecular weight distribution satisfies Condition X,

    Formula (1):        $A\text{-}[(Gly\text{-}X\text{-}Y)_n]_m\text{-}B$

    in the formula, n X's each independently represent any amino acid, n Y's each independently represent any amino acid, m is an integer of 2 to 10, n is an integer of 3 to 100, A represents any amino acid or amino acid sequence, and B represents any amino acid or amino acid sequence,
    Condition X: An area of the maximum molecular weight peak in molecular weight distribution measurement by gel permeation chromatography is 80% or more of the total area of all of the molecular weight peaks.

2. The cell mass or cell structure-embedding agent according to claim 1,
    wherein the polypeptide is recombinant gelatin.

3. The cell mass or cell structure-embedding agent according to claim 1 or 2,
    wherein the polypeptide is polypeptide represented by Formula 2,

    Formula 2:        $Gly\text{-}Ala\text{-}Pro\text{-}[(Gly\text{-}X\text{-}Y)_{63}]_3\text{-}Gly$

in the formula, 63 pieces of X each independently represent any amino acid and 63 pieces of Y each independently represent any amino acid, and 63 pieces of Gly-X-Y may be identical to or different from each other.

4. The cell mass or cell structure-embedding agent according to any one of claims 1 to 3, wherein the polypeptide has (1) an amino acid sequence presented in SEQ ID NO: 1 or (2) an amino acid sequence that has 80% or more of sequence identity with the amino acid sequence presented in SEQ ID NO: 1 and has biocompatibility.

5. The cell mass or cell structure-embedding agent according to any one of claims 1 to 4, wherein the polypeptide has an amino acid sequence presented in SEQ ID NO: 1.

6. A cell mass or cell structure-containing composition comprising:

a cell mass or cell structure; and
the cell mass or cell structure-embedding agent according to any one of claims 1 to 5,
wherein the cell mass or the cell structure is embedded with the cell mass or cell structure-embedding agent.

7. A kit comprising:

a cell mass or cell structure; and
the cell mass or cell structure-embedding agent according to any one of claims 1 to 5.

## FIG. 1

## FIG. 2

# FIG. 3

|  | BEFORE STIRRING | AFTER STIRRING |
|---|---|---|
| WITHOUT 1% CBE3 | 500um | BROKEN PIECES 500um |
| WITH 1% CBE3 | 500um | 500um |

EP 3 590 952 A1

**INTERNATIONAL SEARCH REPORT**

International application No.

PCT/JP2018/007943

A. CLASSIFICATION OF SUBJECT MATTER
Int.Cl.    C07K2/00(2006.01)i,    C12N1/04(2006.01)i,    C12N11/08(2006.01)i,
           C12N15/09(2006.01)n
According to International Patent Classification (IPC) or to both national classification and IPC

B. FIELDS SEARCHED

Minimum documentation searched (classification system followed by classification symbols)
Int.Cl. C07K2/00, C12N1/04, C12N11/08, C12N15/09

Documentation searched other than minimum documentation to the extent that such documents are included in the fields searched
| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS
(STN)

C. DOCUMENTS CONSIDERED TO BE RELEVANT

| Category* | Citation of document, with indication, where appropriate, of the relevant passages | Relevant to claim No. |
|---|---|---|
| Y | JP 8-9966 A (SUMITOMO BAKELITE CO., LTD.) 16 January 1996, claims, examples (Family: none) | 1-7 |
| Y | JP 2010-519251 A (FUJIFILM MANUFACTURING EUROPE B.V) 03 June 2010, claims, paragraphs [0008], [0017], [0018], [0029], [0030], examples & WO 2008/050098 A1, claims, pp. 2, 1.5-21, 4, 1.10-5, 1.7, 7, 1.29-8, 1.15, examples & US 2010/0119574 A1 & EP 2121749 A1 | 1-7 |
| Y | WO 2011/108537 A1 (FUJIFILM CORP.) 09 September 2011, claims, paragraph [0042], examples & US 2013/0004549 A1, paragraph [0041] & EP 2543398 A1 | 1-7 |

☒  Further documents are listed in the continuation of Box C.        ☐  See patent family annex.

| | | |
|---|---|---|
| * | Special categories of cited documents: | |
| "A" | document defining the general state of the art which is not considered to be of particular relevance | "T"  later document published after the international filing date or priority date and not in conflict with the application but cited to understand the principle or theory underlying the invention |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
|---|---|
| 18 May 2018 (18.05.2018) | 29 May 2018 (29.05.2018) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2018/007943

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-183595 A (NITTA GELATIN INC.) 03 July 2003, paragraph [0007] (Family: none) | 1-7 |
| Y | JP 6-242541 A (KONICA CORP.) 02 September 1994, paragraph [0023] (Family: none) | 1-7 |
| Y | 高橋真哉他, ゼラチンのゲル化挙動について 振動式粘度計を用いたゾルゲル変化挙動測定, ゼラチンシンポジウム講演要旨, 1998, vol. 4, pp, 16-20, 17, ("On the gelation behavior of gelatin. Sol gel change behavior measurement with vibratory viscometer."), non-official translation (TAKAHASHI, Shinya et al., Lecture Abstracts Gelatin Symposium) | 1-7 |
| A | JP 2011-172925 A (TERUMO CORP.) 08 September 2011 (Family: none) | 1-7 |
| P, X | WO 2017/170342 A1 (FUJIFILM CORP.) 05 October 2017, claims, examples (Family: none) | 1-7 |

Form PCT/ISA/210 (continuation of second sheet) (January 2015)

## REFERENCES CITED IN THE DESCRIPTION

**Patent documents cited in the description**

- JP 2004357694 A
- WO 2011108517 A
- JP 2005035945 A
- WO 2008103041 A
- EP 1014176 A
- US 6992172 B
- WO 200485473 A
- EP 1014176 A2
- JP 5268933 A
- JP H05268933 A
- WO 2015046216 A1
- JP 18007943 B

**Non-patent literature cited in the description**

- *Pharmaceutical Bulletin,* 1954, vol. 2 (2), 163-173
- *Area of Chemistry,* 1957, vol. 11 (10), 719-725
- *Fragrance Journa,* 1981, vol. 50, 79-82
- **YOSHIO KOUDA.** New Edition Organic Conceptual Diagram -Foundation and Application. Sankyo Shuppan Co., Ltd, 2008
- Protein Identification and Analysis Tools on the Ex-PASy Server. **GASTEIGER E. ; HOOGLAND C. ; GATTIKER A. ; DUVAUD S. ; WILKINS M.R. ; APPEL R.D. ; BAIROCH A.** The Proteomics Protocols Handbook. Humana Press, 2005, 571-607
- **GASTEIGER E. ; GATTIKER A. ; HOOGLAND C. ; IVANYI I. ; APPEL R.D. ; BAIROCH A.** ExPASy: the proteomics server for in-depth protein knowledge and analysis. *Nucleic Acids Res.,* 2003, vol. 31, 3784-3788
- Pathophysiology. Nagai Shoten Co., Ltd, 1990, vol. 9, 527
- **MAXEY, C. R.** Phitogr. Gelatin. 1976, vol. 2
- the Basic Local Alignment Search Tool (BLAST) program. *J. Mol. Biol.,* 1990, vol. 215, 403-410